Europäisches Patentamt

European Patent Office

Office européen des brevets

(11) Veröffentlichungsnummer: **0 279 435 B1**

(12) **EUROPÄISCHE PATENTSCHRIFT**

(45) Veröffentlichungstag der Patentschrift: **05.08.92**

(21) Anmeldenummer: **88102329.5**

(22) Anmeldetag: **18.02.88**

(51) Int. Cl.⁵: **C12P 7/02**

(54) **Verfahren zur Reduktion von Mono- und Dicarbonsäuren.**

(30) Priorität: **19.02.87 DE 3705272**

(43) Veröffentlichungstag der Anmeldung:
**24.08.88 Patentblatt 88/34**

(45) Bekanntmachung des Hinweises auf die
Patenterteilung:
**05.08.92 Patentblatt 92/32**

(84) Benannte Vertragsstaaten:
**CH DE FR GB IT LI NL**

(56) Entgegenhaltungen:

CHEMICAL ABSTRACTS, Band 107, 1987, Seite 393, Zusammenfassung Nr. 150914t, Columbus, Ohio, US; H. WHITE et al.:
"Clostridium thermoaceticum forms methanol from carbon monoxide in the presence
of viologen dyes"

CHEMICAL ABSTRACTS, Band 107, 1987 Seite 668, Zusammenfassung Nr. 175460e, Columbus, Ohio, US; H. SIMON et al.:
"Reduction of 2-enoates and alkanoates with
carbon monoxide or formate, viologens, and
Clostridium thermoaceticum to saturated
acids and unsaturated or saturated alcohols"

CHEMICAL ABSTRACTS, Band 100, 1984, Seite 400, Zusammenfassung Nr. 21536c, Columbus, Ohio, US; H. SIMON et al.: "Chiral
synthons by biohydrogenation or electroenzymic reduction"

(73) Patentinhaber: **BASF Aktiengesellschaft**
**Carl-Bosch-Strasse 38**
**W-6700 Ludwigshafen(DE)**

(72) Erfinder: **Simon, Helmut, Prof. Dr.**
**Egilbertstrasse 31**
**W-8050 Freising(DE)**
Erfinder: **Lebertz, Herbert, Dr.**
**Albert-Sigismund-Strasse 9**
**W-8050 Freising(DE)**

**Beschreibung**

Es ist bereits bekannt, daß biologische Zellen unverzweigte Carbonsäuren sehr unterschiedlicher Kettenlänge zu Alkoholen reduzieren. Entsprechende Umsetzungen wurden mit bakteriellen (J. Biol. Chem. 259, 7109 (1984) und darin zitierte ältere Literatur), pflanzlichen (Meth. Enzymol. 71, 263 (1981)) und tierischen Systemen (J. Biol. Chem. 256, 9542; Biochem. Biophys. Acta 665, 34 (1981)) beschrieben. In allen bisher bekannten Fällen wird dabei nicht das Carboxylat als solches, sondern eine aktivierte Acylverbindung mit NADH oder NADPH reduziert. Nur unter Kopplung mit einer energiereichen Verbindung kann die Reaktion

$$RCOO^- + 2\ NAD(P)H + 3\ H^+ \rightarrow RCH_2OH + 2NAD(P)^+ + H_2O$$

die mit 6,8 kcal/mol endergon ist, bewerkstelligt werden.

Es wurde nun gefunden, daß man Carbonsäuren in einfacher Weise zu den entsprechenden Alkoholen reduzieren kann.

Gegenstand der Erfindung ist ein Verfahren zur mikrobiellen Reduktion von Mono- oder Dicarbonsäuren mit bis zu 10 C-Atomen, die auch Doppelbindungen enthalten und durch Halogenatome, Phenyl- oder Hydroxgruppen substituiert sein können, zu den entsprechenden Alkoholen, welches dadurch gekennzeichnet ist, daß man die Reduktion in Gegenwart eines Mediators mit Kohlenmonoxid und/oder Formiat durchführt.

Als Mono- und Dicarbonsäuren eignen sich insbesondere für die Reaktion: gesättigte einbasische Carbonsäuren - wie Essigsäure, Propionsäure, Buttersäure, 2-Methylbuttersäure, n-Pentansäure, n-Hexansäure -, gesättigte Dicarbonsäuren - wie Bernsteinsäure, Glutarsäure, Adipinsäure, Suberinsäure -, gegebenenfalls alkylsubstituierte Benzoesäure, Phthalsäuren, Terephthalsäure, aliphatisch-aromatische Carbonsäuren - wie 2- und 3-Phenylbuttersäure, Zimtsäure- und Cyclohexancarbonsäure. Als substituierte Carbonsäuren sind insbesondere hydroxylierte und chlorierte Carbonsäuren - wie Milschsäure und 3-Chlorpropionsäure - zu nennen.

Für die Reduktion geeignete Mikroorganismen sind beispielsweise Clostridium thermoaceticum (DSM 521), Clostridium aceticum (DSM 1496), Clostridium formicoaceticum (DSM 92) und Butyribacterium methylotrophicum (DSM 3468). Diese Mikroorganismen eignen sich für die Reduktion besonders gut. Die Reduktion gelingt auch mit Acetobacterium woodii (DSM 1030), Desulfobacterium autotrophicum (DSM 3382) und Eubacterium limosum (DSM 20402). Weitere geeignete Mikroorganismen lassen sich durch einfaches Testen ermitteln.

Die Mikroorganismen können direkt, als Rohextrakte oder in immobilisierter Form für die Reduktion verwendet werden.

Als Mediatoren kommen folgende Substanzen in Betracht:
1. Viologenfarbstoffe, z.B. Methylviologen, Benzylviologen, Diquat,
2. Anthrachinon und andere Chinon-Farbstoffe, z.B. Phenosafranin, Anthrachinonsulfonsäuren,
3. Triphenylmethan-Farbstoffe, z.B. Methylviolett, Kristallviolett,
4. Phthalocyanine, z.B. Fe-, Cu- oder Co-, Phthalocyanin,
5. Methinfarbstoffe, z.B. Astraphloxin,
6. Pyrrolfarbstoffe oder Porphyrinderivate, z.B. Metall-Chelatkomplexe dieser Verbindungen,
7. Pteridine und Pteridone,
8. Flavine, z.B. Acriflavin, Lumiflavin,
9. Metallkomplexe der Metalle der 6., 7., 8. Nebengruppe, z.B. $Ru(L_2L'_2)^{++}$ [L = 1,10 Phenanthrolin, 2,2-Bipyridyl oder 5-Nitro-1, 10-phenanthrolin; L' = Pyridin oder 4-Methylpyridin], 1,1'-Bis(hydroxymethyl)-ferrocene bzw. Ferrocen-Monocarbonsäuren.

Unter diesen ist die 1. Gruppe, insbesondere das Methyl- und Benzylviologen, bevorzugt.

Als Mediatoren können auch bestimmte Metallkomplexe wie Kobaltsepulchrat verwendet werden.

Das neue Verfahren wird vorzugsweise in einem Puffer bei 30 bis 60°C durchgeführt. Zweckmäßigerweise arbeitet man bei einem pH-Wert von etwa 5 bis 6. Das Kohlenmonoxid kann in das Reaktionsgefäß eingeleitet werden Kohlenmonoxid-Atmosphäre.

Reduziert man nach dem neuen Verfahren racemische Säuren, so wird in der Regel ein Antipode des Racemats schneller in den Alkohol umgewandelt als der andere. Man kann deshalb im Endprodukt einen Antipoden stark anreichern, indem man die Reaktion vorzeitig abbricht.

Das neue Verfahren besitzt den Vorteil, daß die Carbonsäuren ohne Aktivierung unter schonenden Bedingungen reduziert werden können, und zwar in annähernd neutralem Bereich und bei Raumtemperatur. Für das Verfahren werden keine Cofaktoren benötigt, die schwer regenerierbar sind.

Beispiel 1

Typische Reduktion von gesättigten Carbonsäuren mit Kohlenmonoxid.

In 1 ml 0,2 M Kaliumphosphatpuffer pH 5,5 wurden 30 mg (Trockengewicht) Clostridium thermoaceticum (DSM 521), 1 mM Methylviologen und 70 $\mu$mol Carbonsäure-Salz gegeben. Unter CO-Atmosphäre wurde der Ansatz bei 40°C geschüttelt. Der Fortgang der Reaktion wurde durch Entnahme kleiner aliquoter Teile durch Gaschromatographie verfolgt. Die Reaktion wurde abgebrochen, wenn keine Carbonsäure mehr nachgewiesen werden konnte.

So wurden aus folgenden Substraten folgende Endprodukte in annähernd quantitativer Ausbeute erhalten:

| Substrat | Produkt |
|---|---|
| Acetat | Ethanol |
| Propionat | 1-Propanol |
| n-Butyrat | 1-Butanol |
| n-Pentanat | 1-Pentanol |
| n-Hexanat | 1-Hexanol |
| Succinat | 1,4-Butandiol |
| Glutarat | 1,5-Pentandiol |
| Adipat | 1,6-Hexandiol |
| Suberinat | 1,8-Octandiol |
| Benzoesäure | Benzylalkohol |
| (2R,S)-Phenylbutyrat | 2-Phenylbutanol |
| (R)-Lactat | 1,2-Propandiol |
| (S)-Lactat | 1,2-Propandiol |
| (E)-2-Methyl-2-butenoat | 2-Methyl-2-butenol |
| (E)-2-Methylzimtat | (2R)-2-Methyl-3-phenyl-propionat |
| Vinylacetat | 3-Butenol-1 |
| Sorbinat | Sorbinol und E-4-Hexenol-1 |

Beispiel 2

Analog Beispiel 1 wurde (R,S)-2-Phenylbutyrat zu 2-Phenylbutanol reduziert. Nach einem Umsatz von 40 % des Ausgangsmaterials wurde die Reaktion abgebrochen. Das so erhaltene 2-Phenylbutanol zeigte in Pentan einen Drehwert von $[\alpha]^D = +10,2°$ (für die reine S-Form wird ein Drehwert von $+16,5°$ angegeben, vgl. Soc. Chim. France 634, 613 (1976)).

Beispiel 3

Zur Immobilisierung von Clostridium thermoaceticum wurden unter Luftausschluß 1,0 g naßgepackte Zellen, aufgeschlämmt in 1,5 ml 0,1 M Kaliumphosphatpuffer pH 7,0, in 5 ml einer 3 %igen Lösung von K-Carrageenan in 0,9 % Natriumchlorid bei 55°C gegeben. Nach kurzem Rühren wurde die Mischung in eine Petrischale von 9 cm Durchmesser gegeben und bei Raumtemperatur erstarren gelassen. Zur weiteren Festigung des Gels wurde es für 30 min mit einer 2 %igen Kaliumchloridlösung überschichtet. Ein Teil dieses Gels, das etwa 0,4 g Zellen enthält, setzt in 23 h unter den in Beispiel 1 angegebenen Bedingungen 84 $\mu$mol Propionat zu Propanol um.

**Patentansprüche**

1. Verfahren zur mikrobiellen Reduktion von Mono- und DicarbonSäuren mit bis zu 10 C-Atomen, die auch Doppelbindungen enthalten und durch Halogenatome, Phenyl- und Hydroxygruppen substituiert sein können, zu den entsprechenden Alkoholen, dadurch gekennzeichnet, daß man die Reduktion in Gegenwart eines Mediators aus der Gruppe

Viologenfarbstoffe, Anthrachinon und andere Chinon-Farbstoffe, Triphenylmethan-Farbstoffe, Phthalocyanine, Methinfarbstoffe, Pyrrolfarbstoffe oder Porphyrinderivate, Pteridine und Pteridone, Flavine, und Metallkomplexe der Metalle der 6., 7. und 8. Nebengruppe

mit Kohlenmonoxid und/oder Formiat durchführt.

**Claims**

1. A process for the microbial reduction of a monocarboxylic or dicarboxylic acid of up to 10 carbon atoms, which may also contain double bonds and be substituted by halogen, phenyl or hydroxyl, to the corresponding alcohol by performing the reduction with carbon monoxide and/or a formate in the presence of a mediator selected from the group consisting of viologen dyes, anthraquinone and other quinone dyes, triphenylmethane dyes, phthalocyanines, methine dyes, pyrrole dyes or porphyrin derivatives, pteridines and pteridones, flavines, and metal complexes of metals of subgroups 6, 7 and 8.

**Revendications**

1. Procédé pour la réduction microbienne des acides monoet di-carboxyliques contenant jusqu'à 10 atomes de carbone, pouvant contenir des doubles liaisons et être substitués par des atomes d'halogènes, des groupes phényle et hydroxy, en les alcools correspondants, caractérisé en ce que l'on procède à la réduction en présence d'un médiateur pris dans le groupe formé par les colorants de viologènes, les colorants d'anthraquinone et d'autres quinones, les colorants du triphénylméthane, les phtalocyanines, les colorants de méthine, les colorants de pyrrole ou les dérivés de porphyrines, les ptéridines et les ptéridones, les flavines et les complexes métalliques des métaux des sous-groupes 6, 7 et 8,

à l'aide de l'oxyde de carbone et/ou d'un formiate.